# EUROPEAN PATENT SPECIFICATION

(11) **EP 3 139 912 B1**
(45) Date of publication and mention of the grant of the patent: **08.04.2020**
(21) Application number: 15789358.7
(22) Date of filing: 06.05.2015
(51) Int. Cl.: A61K 31/192, A61K 31/095, A61K 31/085, A61K 9/00, A61P 11/00

(54) **METHOD OF TREATING IDIOPATHIC PULMONARY FIBROSIS**
VERFAHREN ZUR BEHANDLUNG VON IDIOPATHISCHER LUNGENFIBROSE
PROCÉDÉ DE TRAITEMENT D'UNE FIBROSE PULMONAIRE IDIOPATHIQUE

(30) Priority: 08.05.2014 US 201461990603 P; 02.06.2014 US 201462006692 P; 18.09.2014 US 201414490623
(43) Date of publication of application: 15.03.2017
(60) Divisional application: 20160047.5
(73) Proprietor: MediciNova, Inc., La Jolla, CA 92037 (US)
(72) Inventor: MATSUDA, Kazuko, La Jolla, California 92037 (US); IWAKI, Yuichi, La Jolla, California 92037 (US)
(74) Representative: Nederlandsch Octrooibureau
(86) International application number: PCT/US2015/029449
(87) International publication number: WO 2015/171749

(56) References cited:
- EP-A1- 0 332 109
- WO-A1-2006/134022
- "MN 001: KCA 757, KCA-757, MN-001", DRUGS IN R, ADIS INTERNATIONAL, AUCKLAND, NZ, vol. 8, no. 6, 1 January 2007 (2007-01-01) , pages 400-402, XP009193268, ISSN: 1174-5886
- Kazuko Mastuda: "MN-001 (tipelukast), a Nonselective Phosphodiesterase, 5-lipoxygenase, Leukotriene, Phospholipase C and Thromboxane A2 Inhibitor, Demonstrates Anti-fibrotic Effects in the Bleomycin-induced Idiopathic Pulmonary Fibrosis Mouse", ICLAF 2014 , 20 September 2014 (2014-09-20), 24 September 2014 (2014-09-24), page 27, XP002774886, Retrieved from the Internet: URL:https://www.iclaf.com/Final_ICLAF_Prog ram.pdf [retrieved on 2017-10-19] -& Kazuko Mastuda: "MN-001 (tipelukast), a Nonselective PDE, 5-LO, LT, PLC Inhibitor Demonstrates Anti-Fibrotic Therapeutic Effects in the Bleomycin-inducedPulmonary Fibrosis Mouse Model", MediciNova 18th International Colloquium on Lung and Airway Fibrosis - ICLAF 2014, 20 September 2014 (2014-09-20), 24 September 2014 (2014-09-24), XP002774961, Retrieved from the Internet: URL:http://medicinova.com/wp-content/uploa ds/2017/07/MN-001-IPF-PP-Pres

## Description

### CROSS-REFERENCE TO RELATED APPLICATIONS

This application claims priority to U.S. provisional application no. 61/990,603, filed May 8, 2014, U.S. provisional application no. 62/006,692, filed June 2, 2014, and US Application No. 14/490,623 filed 9/18/2014.

### FIELD

This technology relates to methods of inhibiting or treating pulmonary fibrosis, conditions leading to or arising from it, and/or negative effects of each thereof by administering phenoxyalkylcarboxylic acids such as MN-001 and MN-002.

### BACKGROUND

Pulmonary fibrosis (PF) describes a condition in which the lung tissue becomes thickened, stiff, and scarred. In some cases, the cause of the fibrosis (scarring) can be determined, but in some other cases the cause remains unknown. When there is no known cause for the development of pulmonary fibrosis (and certain radiographic and/or pathologic criteria for pulmonary fibrosis are met), the disease is called idiopathic pulmonary fibrosis or IPF.

There are more than 200 related diseases of the lung known as interstitial lung diseases (ILD), which are also referred to as diffuse parenchymal lung diseases or DPLD. Because these diseases affect the interstitium, the space around the alveoli, ILDs are classified as a group. However, ILDs may also affect other parts of the lungs. Many ILDs have similar characteristics to IPF and most result in lung fibrosis.

A recent study estimates the prevalence of all ILDs in the United States at about 500,000, with IPF being the most common. In the United States, IPF affects from 132,000 to 200,000 people. Approximately 50,000 new cases are diagnosed each year and as many as 40,000 Americans die from IPF each year. There is limited information on the prevalence of IPF in the European Union (EU). The current estimate of the incidence of IPF in the EU is from 37,000 to 40,000 people; in the United Kingdom more than 5,000 new cases are diagnosed each year. It is expected that the number of individuals diagnosed with IPF will continue to increase. This is likely to be a result of people living longer, an improved clinical understanding of IPF, and earlier and more accurate diagnosis.

IPF has no specific demographic profile; it is found in equal proportions in urban and rural environments. A history of smoking and certain genetic factors has been associated with an increased risk of IPF, and a variety of published studies have indicated that about two-thirds of individuals with IPF have a history of smoking. IPF affects more men than women and usually occurs between the ages of 50 and 70.

Document WO 2006/134022 discloses a medicament comprising an MRP4 inhibitor and the LTD4 antagonist MN-001 (compound (IA)) for use in the treatment of interstitial pulmonary diseases, including idiopathic pulmonary fibrosis.

### SUMMARY

The present invention provides a compound of formula (I), an ester of the compound of formula (I), or a pharmaceutically acceptable salt of each of the foregoing for use in a method of treating a patient diagnosed with idiopathic pulmonary fibrosis (IPF), the method comprising administering to the patient an effective amount of the compound, the ester, or the salt as the sole active ingredient; 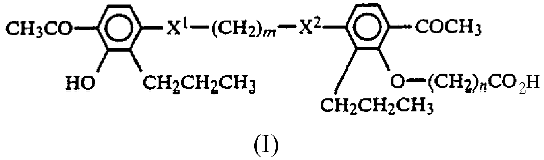 wherein m is an integer from 2 to 5 inclusive, and n is an integer from 3 to 8 inclusive, X¹ and X² each independently represent sulfur, oxygen, a sulfinyl (-S(O)-) group, or a sulfonyl (-S(O)₂-) group, provided that X¹ and X² are not simultaneously oxygen.

In one embodiment the compound of Formula (I) is of Formula (IA):

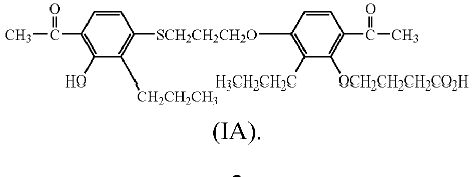

In another aspect of the disclosure the metabolite of the compound of Formula (I) is a compound of Formula (IB): 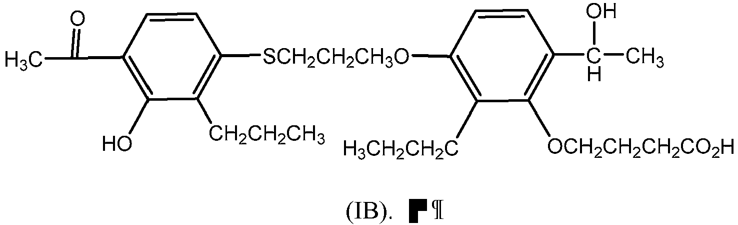 Note that the scope of the invention is defined by the claims. Any references in the description to methods of treatment refer to the compounds, pharmaceutical compositions and medicaments of the present invention for use in a method for treatment of the human (or animal) body by therapy.

Preferably, the compound is administered orally. The compound may be provided as a solid dosage form, such as a tablet or a capsule, and, more preferably, the compound is present in an orthorhombic crystalline polymorphic form. The compound may also be administered as a liquid dosage form. In an aspect of these methods, the compound is administered in an amount ranging from about 100 to about 4,000 mg/day, divided into one, two, or three portions.

In one aspect of these methods, the IPF patient's pulmonary scarring is inhibited. The IPF patient's elevated lung hydroxyproline levels may also be reduced and/or inhibited. At times the IPF patient's elevated lung density is reduced, and in still other times, the IPF patient's elevated total cell count (TCC) in bronchoalveolar lavage fluid (BALF) is reduced.

A method of treating a patient diagnosed with IPF is described, the method comprising administering to the patient an effective amount of a compound of Formula (IA), an ester of the compound of Formula (IA), 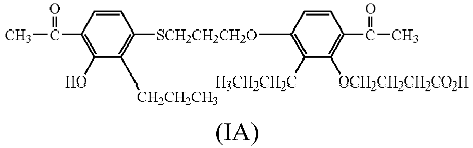 or a pharmaceutically acceptable salt of each of the foregoing.

Also disclosed is a method of treating a patient diagnosed with IPF, the method comprising administering to the patient an effective amount of a compound of Formula (IB), an ester of the compound of Formula (IB): 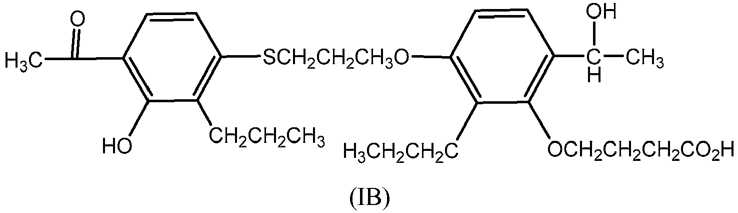 or a pharmaceutically acceptable salt of each of the foregoing.

And still another method provided is one of treating a patient diagnosed with IPF, the method comprising administering to the patient an effective amount of a compound of Formula (IA), an ester of the compound of Formula (IA): 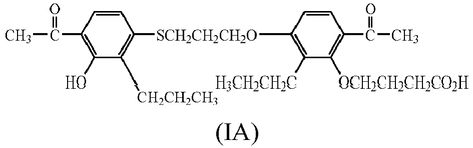 or a pharmaceutically acceptable salt of each of the foregoing, wherein each of the foregoing is provided as a solid dosage form comprising orthorhombic crystals. The solid dosage form is preferably administered orally.

It should be noted that the present disclosure provides methods of treating pulmonary fibrosis, idiopathic pulmonary fibrosis on the one hand and non-idiopathic pulmonary fibrosis on the other.

In another aspect, the present disclosure provides a method of inhibiting pulmonary scarring in a patient in need thereof. The method includes administering to the patient an effective amount of a compound of Formula (I), or an ester thereof, or a pharmaceutically acceptable salt of each thereof, wherein the compound of Formula (I) is defined as above.

In another aspect, the present invention provides a method of reducing and/or inhibiting hydroxyproline formation or collagen formation in a lung of a patient in need thereof. The method includes administering to the patient an effective amount of a compound of Formula (I), or an ester thereof, or a pharmaceutically acceptable salt of each thereof, wherein the compound of Formula (I) is defined as above. In one embodiment, the patient is suffering from pulmonary fibrosis. In another embodiment, the pulmonary fibrosis is idiopathic pulmonary fibrosis. In another embodiment, the pulmonary fibrosis is non-idiopathic pulmonary fibrosis.

In another aspect, the present invention provides a method of reducing elevated lung density in a patient in need thereof. The method includes administering to the patient an effective amount of a compound of Formula (I), or an ester thereof, or a pharmaceutically acceptable salt of each thereof, wherein the compound of Formula (I) is defined as above. In one embodiment, the patient is suffering from pulmonary fibrosis. In another embodiment, the pulmonary fibrosis is idiopathic pulmonary fibrosis. In another embodiment, the pulmonary fibrosis is non-idiopathic pulmonary fibrosis.

In another aspect, the present invention provides a method of reducing elevated total cell count (TCC) in broncholalveolar lavage fluid (BALF) in a patient in need thereof. The method includes administering to the patient an effective amount of a compound of Formula (I), or an ester thereof, or a pharmaceutically acceptable salt of each thereof, wherein the compound of Formula (I) is defined as above. In one embodiment, the patient is suffering from pulmonary fibrosis. In another embodiment, the pulmonary fibrosis is idiopathic pulmonary fibrosis. In another embodiment, the pulmonary fibrosis is non-idiopathic pulmonary fibrosis.

In some embodiments, the methods provided herein are performed in combination with administration of one or more of corticosteroids (such as prednisone), cyclophosphamide, azathioprine, N-acetylcysteine, pirfenidone, and supplemental oxygen therapy.

### BRIEF DESCRIPTION OF FIGURES

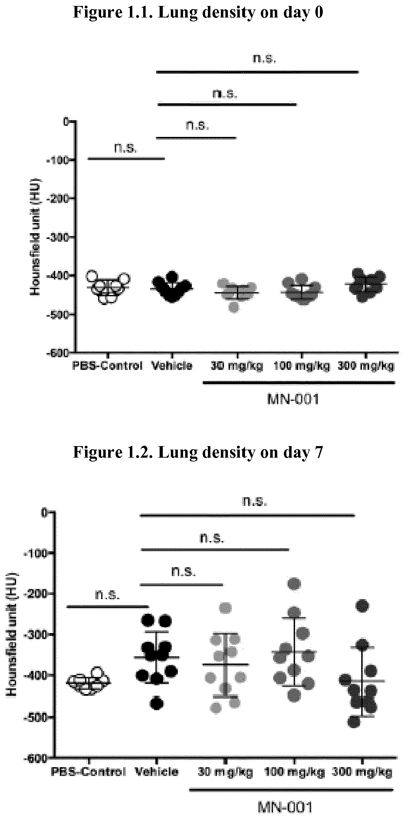

**Figure 1.1** graphically illustrates lung density on day 0 in the control, vehicle and treatment groups.

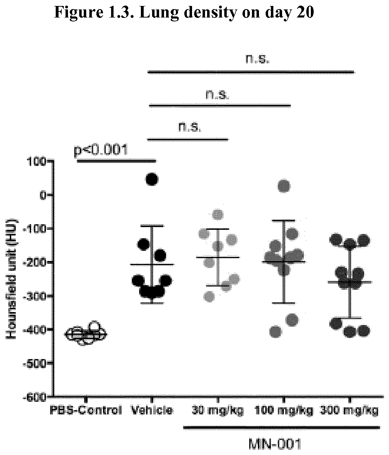

**Figure 1****.****2** graphically illustrates lung density on day 7 in the control, vehicle and treatment groups.
**Figure 1****.****3** graphically illustrates lung density on day 20 in the control, vehicle and treatment groups.
**Figure 2** graphically illustrates lung hydroxyproline content in the control, vehicle and treatment groups.
**Figure 3** graphically illustrates Ashcroft scores in the control, vehicle and treatment groups.

### DETAILED DESCRIPTION

### Definitions

As used herein, and in the appended claims, the singular forms "a," "an" and "the" include plural references unless the context clearly dictates otherwise.

"Administering" or "Administration of' a drug to a patient (and grammatical equivalents of this phrase) includes both direct administration, including self-administration, and indirect administration, including the act of prescribing a drug. For example, as used herein, a physician who instructs a patient to self-administer a drug and/or provides a patient with a prescription for a drug is administering the drug to the patient.

"C_{X}" when placed before a group refers to the number of carbon atoms in that group to be X.

"Alkyl" refers to a monovalent acyclic hydrocarbyl radical having 1 to-12 carbon atoms. Non limiting examples of alkyl include methyl, ethyl, propyl, isopropyl, butyl, isobutyl, tertiary butyl, pentyl, hexyl and the like.

"Aryl" refers to a monovalent aromatic hydrocarbyl radical having up to 10 carbon atoms. Non-limiting examples of aryl include phenyl and naphthyl.

"Heteroaryl" refers to an aromatic group of from 1 to 10 carbon atoms and 1 to 4 heteroatoms selected from the group consisting of oxygen, nitrogen, sulfur within the aromatic ring, wherein the nitrogen and/or sulfur atom(s) of the heteroaryl are optionally oxidized (e.g., N-oxide, -S(O)- or -S(O)₂-). Such heteroaryl groups can have a single ring (e.g., pyridyl or furyl) or multiple condensed rings (e.g., indolizinyl or benzothienyl) wherein the condensed rings may or may not be aromatic and/or contain a heteroatom provided that the point of attachment is through an atom of the aromatic heteroaryl group. Non limiting examples of heteroaryl include pyridyl, pyrrolyl, indolyl, thiophenyl, and furyl.

"Cycloalkyl" refers to a monovalent non-aromatic cyclic hydrocarbyl radical having 3-12 carbon atoms. Non limiting examples of cycloalkyl include cyclopropyl, cyclobutyl, cyclopentyl, cyclohexyl, and the like.

"Heterocyclyl" refers to a monovalent non-aromatic cyclic group of 1 to 10 carbon atoms and 1 to 4 heteroatoms selected from the group consisting of oxygen, nitrogen, sulfur within the cycle, wherein the nitrogen and/or sulfur atom(s) of the heteroaryl are optionally oxidized (e.g., N-oxide, -S(O)- or -S(O)₂-). Such heteroaryl groups can have a single ring (e.g., piperidinyl or tetrahydrofuranyl) or multiple condensed rings wherein the condensed rings may or may not be aromatic and/or contain a heteroatom provided that the point of attachment is through an atom of the non-aromatic heterocyclyl group. Non limiting examples of heterocyclyl include pyrrolidinyl, piperidinyl, piperazinyl, and the like.

"Amino" refers to -NH₂.

"Alkylamino" refers to -NHR_{B}, wherein R_{B} is C₁-C₆ alkyl optionally substituted with 1-3 aryl, heteroaryl, cycloalkyl, or heterocyclyl group.

"Dialkylamino" refers to -N(R_{B})₂, wherein R_{B} is defined as above.

"Comprising" shall mean that the methods and compositions include the recited elements, but not exclude others. "Consisting essentially of' when used to define methods and compositions, shall mean excluding other elements of any essential significance to the combination for the stated purpose. Thus, a composition consisting essentially of the elements as defined herein would not exclude trace contaminants from the isolation and purification method and pharmaceutically acceptable carriers, such as phosphate buffered saline, preservatives and the like. "Consisting of' shall mean excluding more than trace elements of other ingredients and substantial method steps for administering the compositions of this invention or process steps to produce a composition or achieve an intended result. Embodiments defined by each of these transitional terms and phrases are within the scope of this invention.

"Effective amount" of a compound utilized herein is an amount that, when administered to a patient treated as herein, will have the intended therapeutic effect, e.g., alleviation, amelioration, palliation or elimination of one or more manifestations of the medical condition in the patient. The full therapeutic effect does not necessarily occur by administration of one dose (or dosage), and may occur only after administration of a series of doses. Thus, an effective amount may be administered in one or more administrations.

"Pulmonary fibrosis (PF)" describes a condition in which the lung tissue becomes thickened, stiff, and scarred. The alveoli (air sacs) and the blood vessels within the lungs are responsible for delivering oxygen to the body. As lung tissue becomes scarred and thicker, it is more difficult for the lungs to transfer oxygen into the bloodstream. As a result, the brain, heart, and other organs do not get the oxygen they need to function properly. In many cases the cause of the fibrosis (scarring) remains unknown. When there is no known cause for the development of pulmonary fibrosis (but certain radiographic and/or pathologic criteria are met), the disease is referred to as "idiopathic pulmonary fibrosis" or IPF. In certain instances, IPF is characterized by chronic, progressive, fibrosing interstitial pneumonia of unknown cause. In certain instances, IPF affects older adults. In certain instances, IPF is associated with the histopathologic and/or radiologic pattern of UIP (usual interstitial pneumonia).

IPF can also be characterized by alternating areas of normal lung, fibrosis, and interstitial inflammation affecting the peripheral and subpleural parenchyma. Hallmarks of fibrosis include subepithelial myofibroblast/fibroblastic foci and increased deposition of collagen and extracellular matrix. This excess scar tissue causes stiffening of the alveolar walls and a decrease in compliance, which leads to the irreversible loss of total lung capacity and the reduced ability to transport oxygen into the capillaries.

IPF has similar characteristics as that of many interstitial lung diseases (ILDs), many of which result in lung fibrosis. There are more than 200 related diseases of the lung known as ILDs, which are also referred to as diffuse parenchymal lung diseases or DPLD. Because these diseases affect the interstitium, the space around the alveoli, ILDs are classified as a group. However, ILDs may also affect other parts of the lungs.

There is a subgroup of ILDs called idiopathic interstitial pneumonias (IIP), where the lung tissue becomes inflamed and scarring can also occur. As used herein, "pneumonia" is used to describe inflammation and not an infection such as bacterial pneumonia. IIP can be classified into a number of pathological subtypes. These subtypes include usual interstitial pneumonia (UIP), non-specific interstitial pneumonia (NSIP), desquamative interstitial pneumonia (DIP), respiratory bronchiolitis-associated interstitial lung disease (RB-ILD), acute interstitial pneumonia (AIP), cryptogenic organizing pneumonia (COP), and lymphocytic interstitial pneumonia (LIP). IPF is a subtype of IIP, the pathological pattern seen in IPF is substantially that of UIP.

If there is a clear association of the fibrosis with another illness or the lung scarring (fibrosis) is the result of a side effect from a medication or an exposure to an agent known to cause PF, then the cause of the fibrosis may no longer be considered idiopathic, and such fibrosis referred to as non-idiopathic pulmonary fibrosis. PF clearly associated with another disease, such as scleroderma or rheumatoid arthritis, can be referred to as pulmonary fibrosis secondary to scleroderma or secondary to rheumatoid arthritis.

Among the factors contributing to PF, certain non-limiting examples include cigarette smoking, prolonged exposure to occupational or environmental contaminants or dusts, viral or bacterial lung infections, certain medicines, such as some antibiotics, antiarrhythmics, anticonvulsants, chemotherapeutic agents, or therapeutic radiation, acid reflux disease (GERD), and genetic predisposition.

Most PF patients have a gradual worsening of lung function over time, although some remain stable. Some patients may experience episodes of acute worsening of lung function without a clinically apparent infection or other cause; these episodes of acute worsening are called "acute exacerbations." A common symptom is shortness of breath, also known as dyspnea, which many patients describe as a feeling of breathlessness. As the condition progresses and the damage to the lungs becomes more severe, breathlessness may occur with minor physical activity such as showering and getting dressed. About 50% of patients with IPF may have "clubbing" of the fingertips due to a lack of oxygen in the blood. Clubbing is a thickening of the flesh under the fingernails, causing the nails to curve downward. Other common symptoms include, chronic dry, hacking cough, fatigue and weakness, discomfort in the chest, loss of appetite, and unexplained weight loss.

One or more of the following tests are useful for identifying a patient suffering from PF or IPF: mediacal history and physical examination, chest X-Ray, high-resolution computerized tomography (HRCT), pulmonary function tests, pulse oximetry, arterial blood gas (ABG) determination, bronchoscopy, bronchoalveolar Lavage (BAL), aurgical lung biopsy, exercise testing, esophogram, and echocardiogram (ECHO).

In certain instances, IPF can be diagnosed based on three factors: exclusion of other known causes of ILD, the presence of a UIP pattern on high-resolution computed tomography (HRCT) in patients not subjected to surgical lung biopsy, and specific combinations of HRCT and surgical lung biopsy pattern in patients subjected to surgical lung biopsy.

"Pharmaceutically acceptable" refers to non-toxic and suitable for administration to a patient, including a human patient.

"Pharmaceutically acceptable salts" refer to salts that are non-toxic and are suitable for administration to patients. Non-limiting examples include alkali metal, alkaline earth metal, and various primary, secondary, and tertiary ammonium salts. When the ester of the compound of Formula (I) includes a cationic portion, for example, when the ester includes an amino acid ester, the salts thereof can include various carboxylic acid, sulfonic acid, and miner acid salts. Certain non-limiting examples of salts include sodium, potassium , and calcium salts.

"Protecting groups" refer to well-known functional groups which, when bound to a functional group, render the resulting protected functional group inert to the reaction to be conducted on other portions of a compound and the corresponding reaction condition, and which can be reacted to regenerate the original functionality under deprotection conditions. The protecting group is selected to be compatible with the remainder of the molecule. A "carboxylic acid protecting group" protects the carboxylic functionality of the phenoxyalkylcarboxylic acids during their synthesis. Non limiting examples of carboxylic acid protecting groups include, benzyl, p-methoxybenzyl, p-nitrobenzyl, allyl, benzhydryl, and trityl. Additional examples of carboxylic acid protecting groups are found in standard reference works such as Greene and Wuts, Protective Groups in Organic Synthesis., 2d Ed., 1991, John Wiley & Sons, and McOmie Protective Groups in Organic Chemistry, 1975, Plenum Press. Methods for protecting and deprotecting the carboxylic acids disclosed herein can be found in the art, and specifically in Greene and Wuts, supra, and the references cited therein.

"Treating" a medical condition or a patient refers to taking steps to obtain beneficial or desired results, including clinical results. For purposes of the various aspects and embodiments of the present invention, beneficial or desired clinical results include, but are not limited to, reduction, alleviation, or amelioration of one or more manifestations of or negative effects of pulmonary fibrosis, improvement in one or more clinical outcomes, diminishment of extent of fibrosis, delay or slowing of fibrosis progression, amelioration, palliation, or stabilization of the fibrosis state, and other beneficial results described herein.

Provided herein are methods administering an effective amount of a compound of Formula (I): 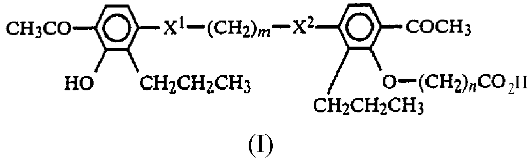 or an ester of the compound of Formula (I) or a pharmaceutically acceptable salt of each thereof, wherein the variables are defined as herein.

As used herein, "an ester thereof' refers to an ester of the phenolic hydroxy group and/or an ester of the carboxylic acid shown in the compound of Formula (I), and an ester of the 1-hydroxyethyl (an aliphatic hydroxy group) group of a metabolite of the compound Formula (I). An ester of the phenolic and/or the aliphatic hydroxy groups can include, without limitation, as the corresponding acid, a carboxylic acid R_{A}-CO₂H, wherein R_{A} is C₁-C₆ alkyl, aryl, heteroaryl, C₃-C₁₂ cycloalkyl, or C₂-C₈ heterocyclyl, wherein the alkyl, aryl, heteroaryl, cycloalkyl, or heterocyclyl are optionally substituted with from 1 to 4 C₁-C₃ alkyl, aryl, CO₂H, amino, alkylamino, or dialkylamino groups. Other acids such as mono-, di-, or tri phosphoric acids are also contemplated. An ester of the carboxylic acid can include, without limitation, as the corresponding alcohol, a compound of formula R_{A}-OH, wherein R_{A} is defined as above. In one embodiment, only the carboxylic acid in Formula (I) is esterified. In another embodiment, only the phenolic hydroxy group in Formula (I) is esterified. In another embodiment, R_{A} is C₁-C₄ alkyl. As will be apparent to the skilled artisan, such esters act as prodrugs that are hydrolyzed *in vivo* to release the compound of Formula (I) or a salt thereof.

In an embodiment, the compound of Formula (I) is a compound of Formula (IA): 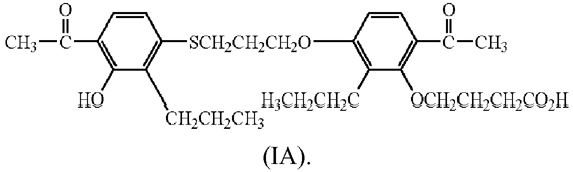 In another aspect of the disclosure a metabolite of the compound of Formula (I) and (IA) is a compound of Formula (IB):

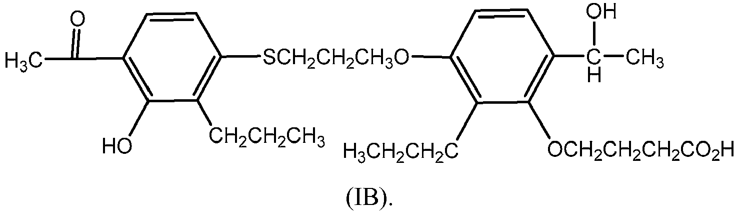

In another embodiment, the compound is administered orally. In another embodiment, the compound is administered as a tablet or a capsule. In another embodiment, the compound of Formula (IA) is present in polymorphic form A that is substantially free of other polymorphic forms. In another embodiment, the compound is administered as a liquid dosage form. In another embodiment, the compound is administered in an amount from about 100 to about 4,000 mg/day, divided into one, two, or three portions.

The efficacy of a compound utilized herein can be tested by methods well known to the skilled artisan, e.g., in the bleomycin induced mouse pulmonary fibrosis model.

### Synthesis

The synthesis and certain biological activity of the compounds of Formula (I) are described in U.S. Pat. No. 4,985,585. For example, the compound of Formula (IA) is prepared by reacting a phenol of Formula (II): 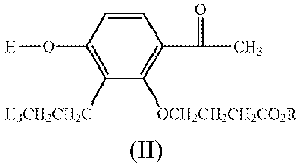 wherein, R is a carboxylic acid protecting group, with a compound of Formula (III): 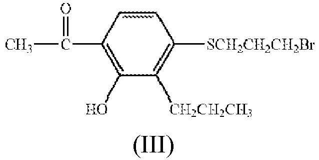 to provide a compound of Formula (IC): 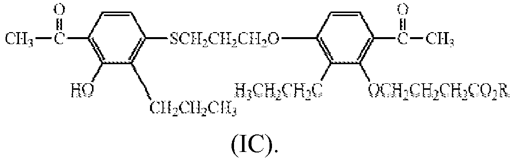 Non-limiting examples of acid protecting groups, or R groups, include C₁-C₆ alkyl, benzyl, benzhydryl, and trityl, wherein the benzyl, benzhydryl, or trityl group is optionally substituted with from 1 to 6 C₁-C₆ alkyl, halo, and/or C₁-C₆ alkoxy groups. It will be apparent to the skilled artisan that a leaving group other than the bromo group of Formula (III) may be used. Non-limiting examples of such other leaving groups include chloro or tosylate.

Deprotection of the protected carboxylic acid of Formula (IC) provides the compound of Formula (IA). As is apparent based on this disclosure, compounds of Formula (IC) are in some embodiments useful in accordance with this invention. Non-limiting examples of deprotection methods include, alkaline hydrolysis and hydrogenolysis under H₂ and a catalyst such as Pd/C or Pt/C.

The reactions are carried out in an inert organic solvent, for example and without limitation, acetone, methylethylketone, diethylketone, or dimethylformamide. The nucleophilic displacement reaction may be conducted at a temperature below room temperature up to the reflux temperature of the solvent, in the presence of an inorganic base, such as potassium carbonate or sodium carbonate, and optionally in the presence of potassium iodide. The reactions are carried out for a period of time sufficient to provide substantial product as determined by well-known methods such as thin layer chromatography and ¹H-NMR. Other compounds utilized herein are made by following the procedures described herein and upon appropriate substitution of starting materials, and/or following methods well known to the skilled artisan. See also, U.S. Pat. No. 5,290,812.

The compound of Formula (IA) is recrystallized under controlled conditions to provide an essentially pure orthorhombic polymorph, referred to as Form A crystals (e.g., 90% or more, preferably at least 95% Form A). Polymorphic Form A and processes for producing it are described in U.S. Pat. Nos. 7,060,854 and 7,064,146. All polymorphic forms of the compound of Formula (I) are active, but polymorphic Form A is preferred. Under certain conditions, the solubility and the bioavailability of this polymorph are superior to the other polymorphs and thus Form A may offer improved solid formulations or solid dosage forms.

Form A crystals can be obtained, For example, by dissolving the compound of Formula (IA) in 5 to 10 parts by weight of ethanol at 25°C to 40°C. to give a yellow to orange solution. The ethanol solution is charged with 1 to 10 parts of water and agitated at 20°C to 25° C for about 15 to 60 minutes and then at 5°C to 10° C. for an additional period of from 1 to 4 hours, preferably 2.0 to 3.0 hours, resulting in an off-white suspension. To this suspension is added 5 to 15 parts of water and the mixture is agitated at 5 to 10° C. for an additional from 1 to 4 hours, preferably 1.5 to 2.0 hours. A solid, white to off-white product is isolated by vacuum filtration and the filter cake is washed with water and dried in a vacuum at 25°C to 40° C for 12 to 24 hours.

For compounds utilized herein that exist in enantiomeric forms, such as certain metabolites of the compound of Formula (I) (for example, the compound of formula IB), the two enantiomers can be optically resolved. Such a resolution is performed, for example, and without limitation, by forming diastereomeric salt of a base such as (S)-(-)-1-(1-naphthyl) ethylamine with the corresponding carboxylic acid compound, or by separating the enantiomers using chiral column chromatography. Intermediates to such compounds, which intermediates also exist in enantiomeric forms can be similarly resolved.

### Administration and Formulation

The compounds utilized herein can be administered orally, or by intravenous, intramuscular, and subcutaneous injection, or transdermal methods. Effective dosage levels can vary widely, e.g., from about 100 to about 4000 mg per day. In one embodiment, the daily dosage range is 250 to 2,000 mg, given in one, two or three portions. In one embodiment, the daily dosage range is 100 to 500 mg, such as 100, 200, 300, 400, or 500 mg given in one, two or three portions. In one embodiment, the daily dosage range is 250 to 2,000 mg, such as 250, 500, 750, 1,000, 1,250, 1,500, 1,750, or 2,000 mg given in one, two or three portions. In one embodiment, the daily dosage range is 1000 to 4,000 mg, such as 1,000, 2,000, 3,000, or 4,000 mg, given in one, two or three portions. In another embodiment, the dosage is 1000 mg twice a day. In other embodiments, suitable dosages include 1000 mg qd, 1000 mg bid, and 750 mg tid.

Actual amounts will depend on the circumstances of the patient being treated. As those skilled in the art recognize, many factors that modify the action of the active substance will be taken into account by the treating physician such as the age, body weight, sex, diet and condition of the patient, the time of administration, the rate and route of administration. Optimal dosages for a given set of conditions can be ascertained by those skilled in the art using conventional dosage determination tests.

The compounds utilized herein can be formulated in any pharmaceutically acceptable form, including liquids, powders, creams, emulsions, pills, troches, suppositories, suspensions, solutions, and the like. Therapeutic compositions containing the compounds utilized herein will ordinarily be formulated with one or more pharmaceutically acceptable ingredients in accordance with known and established practice. In general, tablets are formed utilizing a carrier such as modified starch, alone or in combination with carboxymethyl cellulose (Avicel), for example at about 10% by weight. The formulations are compressed at from 1,000 to 3,000 pounds pressure in the tablet forming process. The tablets preferably exhibit an average hardness of about 1.5 to 8.0 kp/cm² , preferably 5.0 to 7.5 kp/cm². Disintegration time varies from about 30 seconds to about 15 or 20 minutes.

Formulations for oral use can be provided as hard gelatin capsules wherein the therapeutically active compounds utilized herein are mixed with an inert solid diluent such as calcium carbonate, calcium phosphate or kaolin, or as soft gelatin capsules in which the compounds are mixed with an oleaginous medium, e.g., liquid paraffin or olive oil. Suitable carriers include magnesium carbonate, magnesium stearate, talc, sugar, lactose, pectin, dextrin, starch, gelatin, tragacanth, methylcellulose, sodium carboxymethyl cellulose, a low melting wax, cocoa butter, and the like.

The compounds utilized herein can be formulated as aqueous suspensions in admixture with pharmaceutically acceptable excipients such as suspending agents, e.g., sodium carboxymethyl cellulose, methylcellulose, hydroxypropylmethyl cellulose, sodium alginate, polyvinylpyrrolidone, gum tragacanth and gum acacia; dispersing or wetting agents such as naturally occurring phosphatide, e.g., lecithin, or condensation products of an alkaline oxide with fatty acids, e.g., polyoxyethylene stearate, or condensation products of ethylene oxide with long chain aliphatic alcohols, e.g, heptadecaethylene-oxycetanol, or condensation products of ethylene oxide with partial esters derived from fatty acids and a hexitol, e.g., polyoxyethylene sorbitol monoleate or condensation products of ethylene oxide with partial esters derived from fatty acids and hexitol anhydrides, e.g., polyoxyethylene sorbitan monoleate. Such aqueous suspensions can also contain one or more preservatives, e.g., ethyl- or n-propyl-p-hydroxy benzoate, one or more coloring agents, one or more flavoring agents and one or more sweetening agents, such as glycerol, sorbitol, sucrose, saccharin or sodium or calcium cyclamate.

Suitable formulations also include sustained release dosage forms, such as those described in U.S. Pat. Nos. 4,788,055; 4,816,264; 4,828,836; 4,834,965; 4,834,985; 4,996,047; 5,071,646; and, 5,133,974.

Other forms suitable for oral administration include liquid form preparations including emulsions, syrups, elixirs, aqueous solutions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizing, and thickening agents. Solid form preparations may contain, in addition to the active component, colorants, flavors, stabilizers, buffers, artificial and natural sweeteners, dispersants, thickeners, solubilizing agents, and the like.

The compounds utilized herein may be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, for example as solutions in aqueous polyethylene glycol. Examples of oily or nonaqueous carriers, diluents, solvents or vehicles include propylene glycol, polyethylene glycol, vegetable oils (e.g., olive oil), and injectable organic esters (e.g., ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilisation from solution for constitution before use with a suitable vehicle, e.g., sterile, pyrogen-free water.

The compounds utilized herein may be formulated for nasal administration. The solutions or suspensions are applied directly to the nasal cavity by conventional means, for example, with a dropper, pipette or spray. The formulations may be provided in a single or multidose form. The patient can administer an appropriate, predetermined volume of the solution or suspension via a dropper or pipette. A spray may be administered for example by means of a metering atomizing spray pump.

The compounds utilized herein may be formulated for aerosol administration, particularly to the respiratory tract and including intranasal administration. The compound will generally have a small particle size for example of the order of 5 microns or less. Such a particle size may be obtained by means known in the art, for example by micronization. The active ingredient is provided in a pressurized pack with a suitable propellant such as a chlorofluorocarbon (CFC), (for example, dichlorodifluoromethane, trichlorofluoromethane, or dichlorotetrafluoroethane), carbon dioxide or other suitable gases. The aerosol may also contain a surfactant such as lecithin. The dose of drug may be controlled by a metered valve. Alternatively the active ingredients may be provided in a form of a dry powder, for example a powder mix of the compound in a suitable powder base such as lactose, starch, starch derivatives such as hydroxypropylmethyl cellulose and polyvinylpyrrolidine. The powder carrier will form a gel in the nasal cavity. The powder composition may be presented in unit dose form for example in capsules or cartridges of, for example gelatin or blister packs from which the powder may be administered by means of an inhaler.

The compounds utilized herein may be formulated for topical administration to the epidermis as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges including active agents in a flavored base, usually sucrose and acacia or tragacanth; pastilles including the active ingredient in an inert base such as gelatin and glycerin or sucrose and acacia; and mouthwashes including the active ingredient in a suitable liquid carrier.

The compounds utilized herein may be formulated for administration as suppositories. In such a formulation, a low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify.

The compounds utilized herein may be formulated for vaginal administration. Pessaries, tampons, creams, gels, pastes, foams or sprays containing in addition to the active ingredient such carriers as are known in the art to be appropriate.

When desired, formulations can be prepared with enteric coatings adapted for sustained or controlled release administration of the active ingredient. A common type of controlled release formulation that may be used for the purposes of the present invention comprises an inert core, such as a sugar sphere, a first layer, coated with an inner drug-containing second layer, and an outer membrane or third layer controlling drug release from the inner layer.

The cores are preferably of a water-soluble or swellable material, and may be any such material that is conventionally used as cores or any other pharmaceutically acceptable water-soluble or water-swellable material made into beads or pellets. The cores may be spheres of materials such as sucrose/starch (Sugar Spheres NF), sucrose crystals, or extruded and dried spheres typically comprised of excipients such as microcrystalline cellulose and lactose.

The substantially water-insoluble material in the first layer is generally a "GI insoluble" or "GI partially insoluble" film forming polymer (dispersed or dissolved in a solvent). As examples may be mentioned ethyl cellulose, cellulose acetate, cellulose acetate butyrate, polymethacrylates such as ethyl acrylate/methyl methacrylate copolymer (Eudragit NE-30-D) and ammonio methacrylate copolymer types A and B (Eudragit RL30D and RS30D), and silicone elastomers. Usually, a plasticizer is used together with the polymer. Exemplary plasticizers include: dibutylsebacate, propylene glycol, triethylcitrate, tributylcitrate, castor oil, acetylated monoglycerides, acetyl triethylcitrate, acetyl butylcitrate, diethyl phthalate, dibutyl phthalate, triacetin, fractionated coconut oil (medium-chain triglycerides).

The second layer containing the active ingredient may be comprised of the active ingredient (drug) with or without a polymer as a binder. The binder, when used, is usually hydrophilic but may be water-soluble or water-insoluble. Exemplary polymers to be used in the second layer containing the active drug are hydrophilic polymers such as polyvinylpyrrolidone, polyalkylene glycol such as polyethylene glycol, gelatine, polyvinyl alcohol, starch and derivatives thereof, cellulose derivatives, such as hydroxypropylmethyl cellulose (HPMC), hydroxypropyl cellulose, carboxymethyl cellulose, methyl cellulose, ethyl cellulose, hydroxyethyl cellulose, carboxyethyl cellulose, carboxymethyl hydroxyethyl cellulose, acrylic acid polymers, polymethacrylates, or any other pharmaceutically acceptable polymer. The ratio of drug to hydrophilic polymer in the second layer is usually in the range of from 1:100 to 100:1 (w/w).

Suitable polymers for use in the third layer, or membrane, for controlling the drug release may be selected from water insoluble polymers or polymers with pH-dependent solubility, such as, for example, ethyl cellulose, hydroxypropylmethyl cellulose phthalate, cellulose acetate phthalate, cellulose acetate trimellitate, polymethacrylates, or mixtures thereof, optionally combined with plasticizers, such as those mentioned above.

Optionally, the controlled release layer comprises, in addition to the polymers above, another substance(s) with different solubility characteristics, to adjust the permeability, and thereby the release rate, of the controlled release layer. Exemplary polymers that may be used as a modifier together with, for example, ethyl cellulose include: HPMC, hydroxyethyl cellulose, hydroxypropyl cellulose, methylcellulose, carboxymethylcellulose, polyethylene glycol, polyvinylpyrrolidone (PVP), polyvinyl alcohol, polymers with pH-dependent solubility, such as cellulose acetate phthalate or ammonio methacrylate copolymer and methacrylic acid copolymer, or mixtures thereof. Additives such as sucrose, lactose and pharmaceutical grade surfactants may also be included in the controlled release layer, if desired.

Also provided herein are unit dosage forms of the formulations. In such forms, the formulation is subdivided into unit dosages containing appropriate quantities of the active component (e.g., and without limitation, a compound of Formula (I) or an ester thereof, or a salt of each thereof). The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Other suitable pharmaceutical carriers and their formulations are described in Remington: The Science and Practice of Pharmacy 1995, edited by E. W. Martin, Mack Publishing Company, 19th edition, Easton, Pa.

The present invention, thus generally described, will be understood more readily by reference to the following examples, which are provided by way of illustration and are not intended to be limiting of the present invention.

### EXAMPLES

The following abbreviations are used in the examples.
- BAL :: Bronchoalveolar lavage
- BALF :: Bronchoalveolar lavage fluid
- BLM :: Bleomycin
- CT :: Computed tomography
- FLAP :: Five-lipoxygenase activating protein
- IP :: Intraperitoneal
- LTC4 :: Leukotriene C4
- PBS :: Phosphate-buffered saline
- ROI :: Region of interest
- SD :: Standard deviation

### Example 1: Therapeutically beneficial effects of MN-001 in bleomycin (BLM) induced pulmonary fibrosis

Pathogen free 7 weeks old female C57BL/6J mice are obtained from SLC Japan, Inc. At day 0, 40 mice will be induced to develop pulmonary fibrosis by a single intratracheal administration of bleomycin sulfate (BLM, Nippon Khyaku, Japan) in phosphate buffered saline (PBS) at a dose of 3 mg/kg, in a volume of 50 µL per animal using Microsprayer (Penn-Century, USA). BLM-induced pulmonary fibrosis model mice are randomized into 4 groups of 10 mice based on the body weight on the day before the start of treatment. Individual body weight will be measured daily during the experimental period. Survival, clinical signs and behavior of mice will be monitored daily. Computed Tomography(CT) scans will be performed at day 0 (before BLM administration), day 7 (before treatment) and day 20 (after treatment).

### Groups:

Group 1 (PBS, Control): Eight normal mice will be intratracheally administered PBS at a volume of 50 µL without any treatment.
Group 2 (Vehicle): Ten BLM-induced pulmonary fibrosis model mice will be orally administered vehicle (0.3% carboxymethyl cellulose (CMC)) at a volume of 10 mL/kg from day 7 to 20.
Group 3 (MN 001 30 mg/kg): Ten BLM-induced pulmonary fibrosis model mice will be orally administered MN-001 at a dose of 30 mg/kg from day 0 to 20,
Group 4 (MN-001 100 mg/kg): Ten BLM-induced pulmonary fibrosis model mice will be orally administered MN-001 at a dose of 100 mg/kg from day 0 to 20.
Group 5 (MN-001 300 mg/kg): Ten BLM-induced pulmonary fibrosis model mice will be orally administered MN-001 at a dose of 300 mg/k from day 0 to 20.

Mice in all groups will be sacrificed for the following assays at day 21. Analysis of BALF (bronchoalveolar lavage fluid): the cells in BALF will be counted with a hemocytometer.

Biochemical assay: Lung hydroxyproline will be quantified by a hydrolysis method. Histopathological assays for lung sections (according to routine methods): perform Masson's Trichrome staining and estimation of Ashcroft Score. Gene expression assays using total RNA from the lung: real time RT-PCR analyses can be performed for TIMP-1, Collagen Type 1, α-SMA, 5-Lipooxygenase, FLAP, and LTC4 synthase. Statistical tests: Statistical tests will be performed using Bonferroni Multiple Comparison Test. Survival curve will be established using the Kaplan-Meier survival method and compared using the Log Rank test. P values < 0.05 can be considered statistically significant.

### Example 2: Treatment of idiopathic pulmonary fibrosis

A randomized, double-blind, placebo-controlled study is performed on 40 patients with idiopathic pulmonary fibrosis. The patients are randomized to receive MN-001 or MN-002 (each at a daily dose of 500 mg for up to 6 months) or placebo.

Patients undergo the following measurements: 1) change from baseline to week 12 in 6-minute walk distance (6MWD), 2) change from baseline to week 12 in hemodynamic parameters (RHC) at rest, and 3) New York Heart Association (NYHA) class from baseline to week 12. Other measurements provided in this study are: 1) change from baseline O₂ desaturation and quantity of desaturation measures during 6MWD at week 6 and 12, 2) change from baseline forced vital capacity (FVC) and diffusing capacity (DLCO) at weeks 6 and 12, 3) change from baseline in dyspnea using Borg scale at weeks 6 and 12, and 4) change from baseline to week 12 in hemodynamics (RHC) at exercise using cycle geometry.

As part of standard of care, the following procedures are performed on subjects: right heart catheterization, transthoracic echocardiogram, 6 minute walk, full pulmonary function tests, HRCT chest and a battery of blood tests (B-type natriuretic peptide (BNP), DDimer, CRP, Troponin I, and liver function testing).

Standard of care blood work is done on the day of catheterization testing for BNP, C reactive protein, D-Dimer, Troponin-I, and liver function testing. Study blood work includes 4 cc of blood into each of four tubes including dark green, purple, red and yellow tops. Blood work (both standard of care and study blood) is repeated on a scheduled basis for all patients enrolled into the study.

Borg Dyspnea Score is measured at the initial 6 MW and with subsequent 6MW done per scheduled (listed below) thereafter. NYHA functional class is determined at the initiation into the study and as scheduled thereafter.

**DATA TIME POINTS FLOW DIAGRAM**

| Initial | 6 weeks | 12 weeks | 6 months | 1 Year |
|---|---|---|---|---|
| R heart cath | | | | R heart cath |
| 6 MW TTE | 6 MW | 6 MW | 6 MW | 6 MW TTE |
| Dyspnea | Dyspnea | Dyspnea | Dyspnea | Dyspnea |
| Score | Score | Score | Score | Score |
| Blood | | Blood | | Blood |
| work (lab) | | work (lab) | | work (lab) |
| QOL | | | | QOL |
| index | | | | index |
| Spirometer | | Spirometer | | Spirometer |
| DLCO | | DLCO | | DLCO |
| (lab) | | (lab) | | (lab) |
| NYHA/ | | | | NYHA/ |
| WHO | | | | WHO |
| class | | | | class |
| HRCT | | | | HRCT |
| chest | | | | chest |

### Example 3: Treatment of idiopathic pulmonary fibrosis and various symptoms thereof

This example demonstrates the effects of MN-001 in Bleomycin (BLM)-induced pulmonary fibrosis. As evidenced by Ashcroft score and lung hydroxyproline content, pulmonary fibrosis was established in all BLM-treated mice. MN-001 treatment showed a significant reduction or a decreasing trend in the Ashcroft score and lung hydroxyproline content compared to the Vehicle group at day 21. In the present study, MN-001 was administered at day 7 after BLM administration. Since BLM induced fibrogenic reaction already at day 7, the treatment schedule in the present study is considered to be a "therapeutic regimen". The treatment with MN-001 has a significant anti-fibrogenic effect when administered after detecting fibrosis in BLM-induced pulmonary fibrosis in mice.

### MATERIALS AND METHODS

### Test substance

To prepare dosing solution, MN-001 was weighed and dissolved in vehicle (0.3% CMC).

### Animals

Seven-week-old female C57BL/6 mice (17∼21 g) were obtained from Japan SLC (Japan). Animals were housed and fed with normal diet (CE-2; CLEA Japan) under conventional conditions. All animals used in this study were cared following appropriate guidelines.

### Environment

The animals were maintained in an animal facility under conventional conditions.

### Animal housing

The animals were housed in polycarbonate cages KN-600 (Natume Seisakusho, Japan) with a maximum of 5 mice per cage. Sterilized Paper-Clean (Japan SLC) was used for bedding for animals and replaced once a week.

### Food and drink

Sterilized normal diet was provided *ad libitum*, being placed in a metal lid on the top of the cage and on the floor to allow easy access. Distilled water was also provided *ad libitum* from a water bottle equipped with a rubber stopper and a sipper tube. Water bottles were replaced once weekly, cleaned, sterilized in an autoclave and reused.

### Animal and cage identification

Mice were identified by numbers engraved on earrings. Each cage was also given a specific identification code.

### Preparation and randomization of BLM-induced pulmonary fibrosis model

On day 0, twenty mice were intratracheally administered BLM (Nippon Kayaku, Japan) in 0.9% saline in a volume of 50 µL per animal using a Microsprayer® (Penn-Century, USA).

### Routes of drug administration

MN-001 and vehicle were administered by oral route in a volume of 10 mL/kg.

### Treatment dose

MN-001 was administered at doses of 30, 100, 300 mg/kg once daily.

### CT evaluation

CT scans were performed at day 0 (before BLM administration), day 7 and day 20 (the day before sacrifice). The mice were mounted on a holder and placed in the X-ray CT system (LCT-200, Aloka, Japan) under pentobarbital sodium (Kyoritu Seiyaku, Japan) anesthesia. The images were converted into DICOM format and analyzed with Onis Viewer (DigitalCore, Japan). Two section slides (upper: forth dorsal vertebra, lower: seventh dorsal vertebra) were determined from each scan data set, and eight regions of interest (ROI) were defined in the following areas: the right upper anterior and posterior regions, the left upper anterior and posterior region. The means of the intensity of the eight ROIs were defined as an individual's level of lung density.

### BALF collection and analyses

BALF samples were collected by flushing the lung via the trachea with sterile PBS three times (0.8 mL each). The first lavage was kept separate from the other two. BALF was centrifuged at 1,000 xg for 3 minutes at 4°C and the supernatant was collected and stored at -80°C until use. The cell pellet from the first fraction and the remaining fractions of lavage fluid were pooled. Total cell number of BALF was counted with a hemocytometer.

### Measurement of lung hydroxyproline content

To quantify lung hydroxyproline content, frozen left lung samples (15-25 mg) were processed by an alkaline-acid hydrolysis method as follows. Lung samples were acid-hydrolyzed with 400 µL of 6N HCl at 121°C for 20 minutes, and neutralized with 400 µL of 4N NaOH containing 10 mg/mL activated carbon. AC buffer (2.2M acetic acid/0.48M citric acid, 400 µL) was added to the samples, followed by centrifugation to collect the supernatant. A standard curve of hydroxyproline was constructed with serial dilutions of trans-4-hydroxy-L-proline (Sigma-Aldrich, USA) starting at 16 µg/mL. The prepared samples and standards (each 400 µL) were mixed with 400 µL chloramine T solution (Wako Pure Chemical Industries Japan) and incubated for 25 minutes at room temperature. The samples were then mixed with Ehrlich's solution (400 µL) and heated at 65°C for 20 minutes to develop the color. After samples were cooled on ice and centrifuged to remove precipitates, the optical density of each supernatant was measured at 560 nm. The concentrations of hydroxyproline were calculated from the hydroxyproline standard curve. Lung hydroxyproline levels were expressed as µg per left lung.

### Histopathological analyses

Right lung tissues prefixed in 10% neutral buffered formalin were embedded in paraffin and sectioned at Masson's trichrome staining, the sections were stained with Masson's trichrome staining Kit (Sigma, USA) according to the manufacturer's instructions The degree of pulmonary fibrosis was evaluated using the Ashcroft score (Ashcroft, T., et al., J Clin Pathol, 1988;41:467-70) for the quantitative histological analysis.

### Statistical tests

Statistical analyses were performed using Bonferroni multiple comparison test on GraphPad Prism 4 (GraphPad Software, USA). P values < 0.05 were considered statistically significant. A trend or tendency was assumed when a one-tailed t-test returned P values < 0.10. Results were expressed as mean ± SD.

### EXPERIMENTAL DESIGN AND TREATMENT

### Treatment groups

### Group 1 (PBS-Control):

Eight normal mice were intratracheally administered PBS at a volume of 50 µl without any treatment.

### Group 2 (Vehicle):

Ten BLM-induced pulmonary fibrosis model mice were orally administered vehicle (0.3% CMC) at a volume of 10 ml/kg from day 7 to 20.

### Group 3 (MN-001 30 mg/kg):

Ten BLM-induced pulmonary fibrosis model mice were orally administered MN-001 at a dose of 30 mg/kg from day 7 to 20.

### Group 4 (MN-001 100 mg/kg):

Ten BLM-induced pulmonary fibrosis model mice were orally administered MN-001 at a dose of 100 mg/kg from day 7 to 20.

### Group 5 (MN-001 300 mg/kg):

Ten BLM-induced pulmonary fibrosis model mice were orally administered MN-001 at a dose of 300 mg/kg from day 7 to 20.
Table 1 below summarizes the treatment schedule.

**Table 1. Treatment schedule**

| **Group** | **No. mice** | **Mice** | **Test substance** | **Dose (mg/kg)** | **Volume (mL/kg)** | **Regimens** | **Sacrifice (Day)** |
|---|---|---|---|---|---|---|---|
| 1 | 8 | Control | - | - | - | - | 21 |
| 2 | 10 | BLM | Vehicle | - | 10 | Oral, once daily, Day 7 to 20 | 21 |
| 3 | 10 | BLM | MN-001 | 30 | 10 | Oral, once daily, Day 7 to 20 | 21 |
| 4 | 10 | BLM | MN-001 | 100 | 10 | Oral, once daily, Day 7 to 20 | 21 |
| 5 | 10 | BLM | MN-001 | 300 | 10 | Oral, once daily, Day 7 to 20 | 21 |

### Animal monitoring and sacrifice

The viability, clinical signs and behavior were monitored every day. Body weight was recorded daily after the day of starting the BLM administration (day 0). Animals were sacrificed by exsanguination through the abdominal aorta under pentobarbital sodium anesthesia (Kyoritsu, Japan).

### RESULTS

### Body weight changes and general condition

Body weight was expressed as percentage body weight change from baseline (day 0).

In the PBS-Control group, mean body weight was gradually increased through the study period. Mean body weight loss was significantly greater in the Vehicle group compared to the PBS-Control group from day 9 to day 20. There were no significant differences in the body weight changes at any day between the Vehicle group and any of the treatment groups.

In the mean body weight loss on the day of sacrifice, there were no significant differences between the Vehicle group and any of the PBS-Control, MN-001 treated groups (PBS-Control: 112.4 ± 4.5%, Vehicle: 106.9 ± 6.4%, MN-001 30 mg/kg: 104.8 ± 3.7%, MN-001 100 mg/kg: 100.9 ± 10.6%, MN-001 300 mg/kg: 104.2 ± 4.1%) (Table 2).

**Table 2. Body weight changes on the day of sacrifice**

| Parameter | PBS-Control | Vehicle | MN-001 30 mg/kg | MN-001 100 mg/kg | MN-001 300 mg/kg |
|---|---|---|---|---|---|
| mean ± SD | (n=8) | (n=8) | (n=8) | (n=10) | (n=10) |
| Body weight changes (%) | 112.4 ± 4.5 | 106±6.4 | 104.8 ± 3.7 | 100.9 ± 10.6 | 104.2 ± 4.1 |

### Survival analysis

In the Vehicle group, two out of 10 mice died during the experimental period. There were no significant differences in the survival rate between the Vehicle group and any of the PBS-Control, MN-001 treated groups.

During the treatment period, mice died before reaching day 21 as follows; two out of 10 mice died in the Vehicle group. Two out of 10 mice died in the MN-001 30 mg/kg group.

### CT analysis

Lung density of the Vehicle group was significant increased on day 21 compared to the PBS-Control group. Though there were no significant differences in the lung density on day 0, 7 and 21 between the Vehicle group and any of the MN-001 treated groups (Table 3) a downward trend in lung density was visually observed in the corresponding graphs (see, Figures 1.1 to 1.3).

**Table 3. CT evaluation**

| Parameter | PBS-Control | Vehicle | MN-001 30 mg/kg | MN-001 100mg/kg | MN-001 100 300mg/kg |
|---|---|---|---|---|---|
| mean ± SD | (n=8) | (n=8) | (n=8) | (n=10) | (n=10) |
| Long density on day 0 | -432 ± 21 | -435 ± 17 | -445 ± 17 | -423 ± 18 | -423 ± 19 |
| Long density on day 7 | -420 ± 13 | -357 ± 63 | -375 ± 77 | ± 84 | -415 ± 83 |
| Long density on day 21 | -415 ± 11 | -207 ± 115 | -186 ± 84 | -200 ± 122 | -260 ± 107 |

### Cellular analysis of BALF analysis

The total number of cells in BALF in the Vehicle group tended to increase compared to the PBS-Control group. There were no significant differences in the total number of cells between the Vehicle group and any MN-001 treated groups (PBS-Control: 3.4 ± 1.0 (x10⁵ cells), Vehicle: 82.9 ± 63.0 (x10⁵ cells), MN-001 30 mg/kg: 65.1 ± 26.1 (x 10⁵ cells), MN-001 100mg/kg: 124.4 ± 127.3 (x10⁴ cells), MN-001 300mg/kg: 76.0 ± 35.0 (x10⁵ cells)).

### Lung hydroxyproline contents

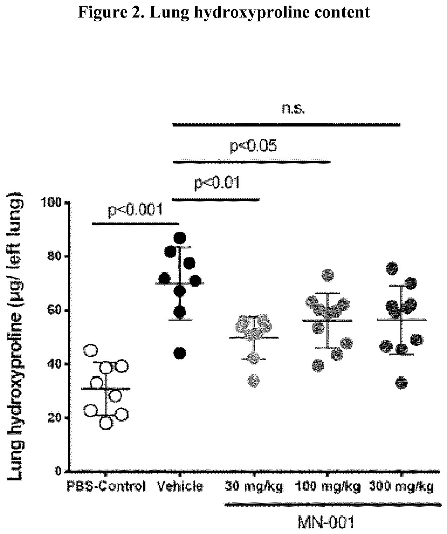

Lung hydroxyproline contents tended to increase in the Vehicle group compared to the PBS-Control group. Lung hydroxyproline contents in the MN-001 30 mg/kg and 100 mg/kg groups significantly decreased compared to the Vehicle group. MN-001 300 mg/kg treatment also tended to decrease the lung hydroxyproline content compared to the Vehicle group (PBS-Control: 30.8 ± 9.8 µg/left lung, Vehicle: 70.0 ± 13.5 µg/left lung, MN-001 30 mg/kg: 49.8 ± 7.9 µg/left lung, MN-001 100 mg/kg: 56.1 ± 10.1 µg/left lung, MN-001 300 mg/kg: 56.4 ± 12.7 µg/left lung). (Figure 2 and Table 4).

**Table 4. Lung hydroxyproline content**

| Parameter | PBS-Control | Vehicle | MN-001 300 mg/kg | MN-001 100 mg/kg | MN-001 300 mg/kg |
|---|---|---|---|---|---|
| mean ± SD | (n=8) | (n=8) | (n=8) | (n=10) | (n=10) |
| Lung Hyp (µg/mg left lung) | 30.8 ± 0.8 | 700 ± 13.5 | 49.8 ±7.9 | 58.1 ± 10.1 | 56.4 ± 12.7 |

### Histological analysis

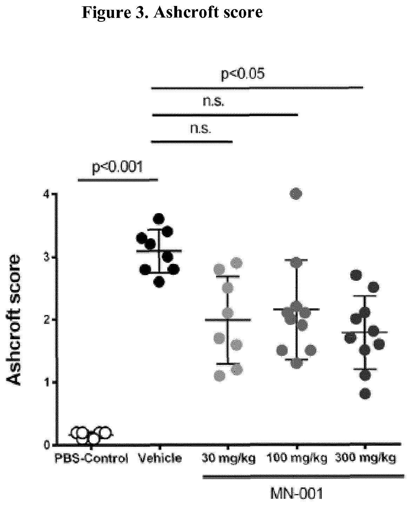

Based on Masson's trichrome staining and Ashcroft score, the Ashcroft score was significantly higher in the Vehicle group compared to the PBS-Control group Ashcroft score in the MN-001 300 mg/kg group significantly decreased compared to the Vehicle group. MN-001 30 mg/kg and 100 mg/kg treatment tended to decrease the Ashcroft score compared to the Vehicle group (PBS-Control: 0.2 ± 0.1, Vehicle: 3.1 ± 0.3, MN-001 30 mg/kg: 2.0± 0.7, MN-001 100 mg/kg: 2.2 ± 0.8, MN-001 300 mg/kg: 1.8 ± 0.6). (Table 5 and Figure 3).

**Table 5. Histopathological analysis**

| Parameter | PBS-Control | Vehicle | MN-001 30 mg/kg | MN-001 100 mg/kg | MN-001 300 mg/kg |
|---|---|---|---|---|---|
| mean ± SD | (n=8) | (n=8) | (n=8) | (n=10) | (n=10) |
| Ashcroft score | 0.2 ± 0.1 | 3.1 ± 0.3 | 2,0 ± 0.7 | 2.2 ± 0.8 | 1.8 ± 0.6 |

While certain embodiments have been illustrated and described, it should be understood that changes and modifications can be made therein in accordance with ordinary skill in the art without departing from the technology in its broader aspects as defined in the following claims.

The embodiments, illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the claimed technology. Additionally, the phrase "consisting essentially of' will be understood to include those elements specifically recited and those additional elements that do not materially affect the basic and novel characteristics of the claimed technology. The phrase "consisting of' excludes any element not specified.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member.

Other embodiments are set forth in the following claims.

## Claims

1. A compound of Formula (I): 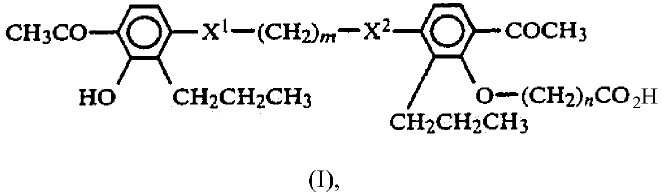 an ester of the compound of Formula (I), or a pharmaceutically acceptable salt of each of the foregoing, for use in a method of treating a patient diagnosed with idiopathic pulmonary fibrosis (IPF),
wherein the method comprises administering to the patient an effective amount of the compound, the ester, or the salt as the sole active ingredient; and
wherein m is an integer from 2 to 5 inclusive, and n is an integer from 3 to 8 inclusive, X¹ and X² each independently represent sulfur, oxygen, a sulfinyl group or a sulfonyl group, provided that X¹ and X² are not simultaneously oxygen.

2. The compound, ester, or salt for use of Claim 1, in which the compound of Formula (I) is of Formula (IA) 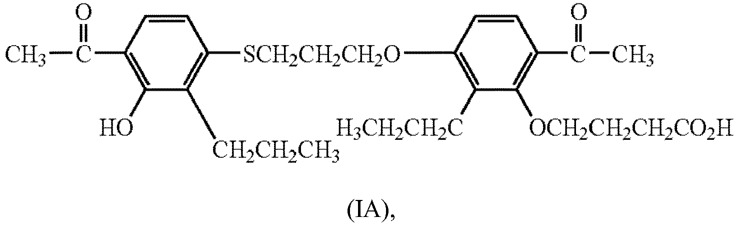 preferably wherein the compound is present in an orthorhombic crystalline polymorphic form.

3. The compound, ester, or salt for use of Claim 1 or 2, wherein the compound is administered orally, preferably wherein the compound is administered as a tablet or a capsule.

4. The compound, ester, or salt for use of Claim 1 or 2, wherein the compound is administered as a liquid dosage form.

5. The compound, ester, or salt for use of Claim 1 or 2, wherein the compound is administered in an amount from about 100 to about 4,000 mg/day, divided into one, two, or three portions.

6. The compound, ester, or salt for use of Claim 1 or 2, wherein the patient's pulmonary scarring is inhibited.

7. The compound, ester, or salt for use of Claim 1 or 2, wherein the patient's elevated lung hydroxyproline levels are reduced and/or inhibited.

8. The compound, ester, or salt for use of Claim 1 or 2, wherein the patient's elevated lung density is reduced.

9. The compound, ester, or salt for use of Claim 1 or 2, wherein the patient's elevated total cell count (TCC) in bronchoalveolar lavage fluid (BALF) is reduced.

10. A compound of Formula (IA): 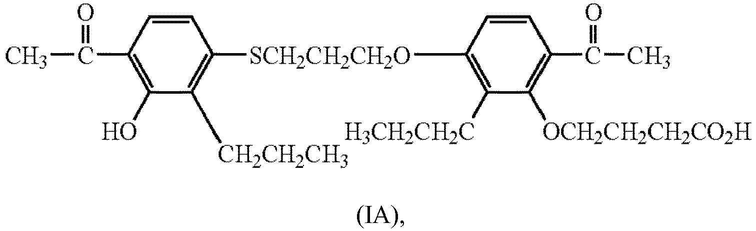 an ester of the compound of Formula (IA), or a pharmaceutically acceptable salt of each of the foregoing, for use in a method of treating a patient diagnosed with IPF, wherein the method comprises administering to the patient an effective amount of the compound, the ester, or the salt as the sole active ingredient, wherein each of the foregoing is provided as a solid dosage form comprising orthorhombic crystals, preferably wherein the compound is administered in an amount from about 100 to about 4,000 mg/day, divided into one, two, or three portions.

11. The compound, ester, or salt for use of Claim 10, wherein the patient's pulmonary scarring is inhibited, or wherein the patient's elevated lung hydroxyproline levels are reduced and/or inhibited, or wherein the patient's elevated lung density is reduced, or wherein the patient's elevated total cell count (TCC) in bronchoalveolar lavage fluid (BALF) is reduced.

12. The compound, ester, or salt for use of Claim 10, wherein the solid dosage form is administered orally.

## Patentansprüche

1. Verbindung nach Formel (I): 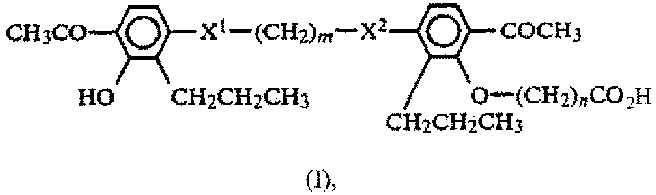 Ester der Verbindung nach Formel I oder pharmazeutisch verträgliches Salz von einem der vorhergehenden, zur Verwendung bei einem Verfahren zum Behandeln eines mit idiopathischer Lungenfibrose (IPF) diagnostizierten Patienten,
wobei das Verfahren umfasst, dem Patienten eine wirksame Menge der Verbindung, des Esters oder das Salzes als einzigen wirksamen Inhaltsstoff zu verabreichen, und
wobei m eine ganze Zahl von 2 bis einschließlich 5 ist und n eine ganze Zahl von 3 bis einschließlich 8 ist, X1 und X2 unabhängig voneinander jeweils Schwefel, Sauerstoff, ein Sulfinylrest oder ein Sulfonylrest sind, unter der Voraussetzung, dass X1 und X2 nicht gleichzeitig Sauerstoff sind.

2. Verbindung, Ester oder Salz zur Verwendung nach Anspruch 1, wobei es sich bei der Verbindung nach Formel (I) um eine nach Formel (IA) handelt 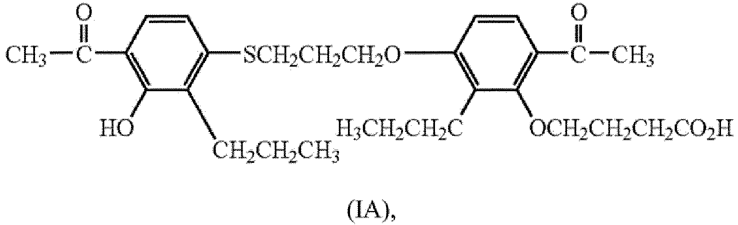 wobei die Verbindung vorzugsweise in einer orthorhombisch-kristallinen polymorphen Form vorliegt.

3. Verbindung, Ester oder Salz zur Verwendung nach Anspruch 1 oder 2, wobei die Verbindung oral verabreicht wird, wobei die Verbindung vorzugsweise als Tablette oder Kapsel verabreicht wird.

4. Verbindung, Ester oder Salz zur Verwendung nach Anspruch 1 oder 2, wobei die Verbindung als flüssige Dosierungsform verabreicht wird.

5. Verbindung, Ester oder Salz zur Verwendung nach Anspruch 1 oder 2, wobei die Verbindung in einer Menge von etwa 100 bis etwa 4.000 mg/Tag, verteilt auf eine, zwei oder drei Portionen, verabreicht wird.

6. Verbindung, Ester oder Salz zur Verwendung nach Anspruch 1 oder 2, wobei die Narbenbildung der Lunge des Patienten inhibiert ist.

7. Verbindung, Ester oder Salz zur Verwendung nach Anspruch 1 oder 2, wobei die erhöhten Lungen-Hydroxyprolinspiegel des Patienten reduziert und/oder inhibiert sind.

8. Verbindung, Ester oder Salz zur Verwendung nach Anspruch 1 oder 2, wobei die erhöhte Lungendichte des Patienten reduziert ist.

9. Verbindung, Ester oder Salz zur Verwendung nach Anspruch 1 oder 2, wobei die erhöhte Gesamtzellzahl (TCC) des Patienten in der bronchoalveolären Lavage-Flüssigkeit (BALF) reduziert ist.

10. Verbindung nach Formel (IA): 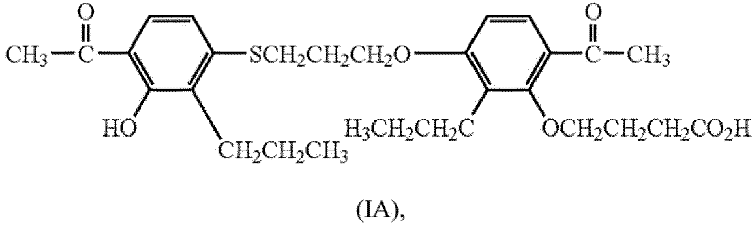 Ester der Verbindung nach Formel (IA) oder pharmazeutisch verträgliches Salz von einem der vorhergehenden, zur Verwendung bei einem Verfahren zum Behandeln eines mit IPF diagnostizierten Patienten, wobei das Verfahren umfasst, dem Patienten eine wirksame Menge der Verbindung, des Esters oder das Salzes als einzigen wirksamen Inhaltsstoff zu verabreichen, wobei jedes der vorhergehenden als feste Dosierungsform, umfassend orthorhombische Kristalle, bereitgestellt wird, wobei die Verbindung vorzugsweise in einer Menge von etwa 100 bis etwa 4.000 mg/Tag, verteilt auf eine, zwei oder drei Portionen, verabreicht wird.

11. Verbindung, Ester oder Salz zur Verwendung nach Anspruch 10, wobei die Narbenbildung der Lunge des Patienten inhibiert ist oder wobei die erhöhten Lungen-Hydroxyprolinspiegel des Patienten reduziert und/oder inhibiert sind oder wobei die erhöhte Lungendichte des Patienten reduziert ist oder wobei die erhöhte Gesamtzellzahl (TCC) des Patienten in der bronchoalveolären Lavage-Flüssigkeit (BALF) reduziert ist.

12. Verbindung, Ester oder Salz zur Verwendung nach Anspruch 10, wobei die feste Dosierungsform oral verabreicht wird.

## Revendications

1. Un composé de formule (I): 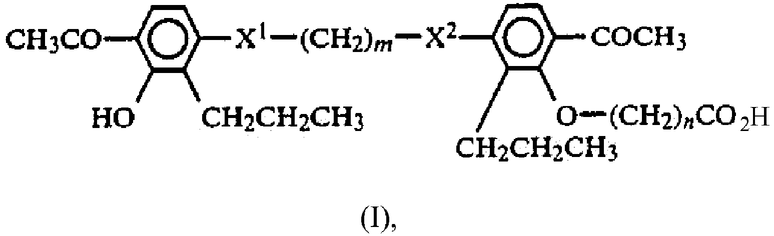 un ester de ce composé de formule (I) ou un sel pharmaceutiquement acceptable de l'un ou l'autre d'entre eux, destiné à être utilisé dans une méthode de traitement d'un patient atteint de fibrose pulmonaire idiopathique (IPF),
- dans lequel la méthode comprend l'administration au patient d'une quantité efficace du composé, de l'ester ou du sel en tant que seul ingrédient actif; et
- dans lequel m est un nombre entier de 2 à 5 inclus, et n est un nombre entier de 3 à 8 inclus, X¹ et X² représentent chacun, indépendamment l'un de l'autre, un atome de soufre, un atome d'oxygène, un groupe sulfinyle ou un groupe sulfonyle, à condition que X¹ et X² ne soient pas simultanément un atome d'oxygène.

2. Le composé, ester ou sel destiné à être utilisé selon la revendication 1, dans lequel le composé de formule (I) présente la formule (IA), 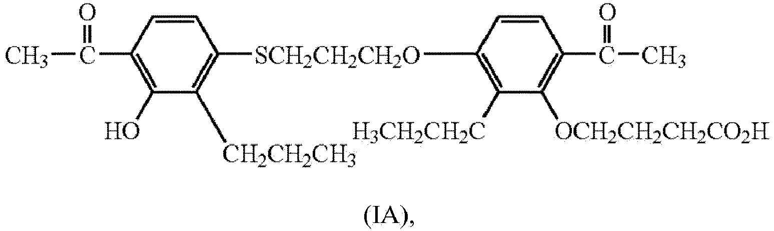 de préférence, le composé est présent sous une forme polymorphique cristalline orthorhombique.

3. Le composé, ester ou sel destiné à être utilisé selon la revendication 1 ou 2, lequel composé est administré par voie orale, de préférence le composé est administré sous forme de comprimé ou de gélule.

4. Le composé, ester ou sel destiné à être utilisé selon la revendication 1 ou 2, lequel composé est administré sous forme de dose liquide.

5. Le composé, ester ou sel destiné à être utilisé selon la revendication 1 ou 2, lequel composé est administré en une quantité d'environ 100 à environ 4 000 mg/jour, divisée en une, deux ou trois portions.

6. Le composé, ester ou sel destiné à être utilisé selon la revendication 1 ou 2, dans lequel la cicatrisation pulmonaire du patient est inhibée.

7. Le composé, ester ou sel destiné à être utilisé selon la revendication 1 ou 2, dans lequel les niveaux élevés d'hydroxyproline dans les poumons du patient sont réduits et/ou inhibés.

8. Le composé, ester ou sel destiné à être utilisé selon la revendication 1 ou 2, dans lequel la densité pulmonaire élevée du patient est réduite.

9. Le composé, ester ou sel destiné à être utilisé selon la revendication 1 ou 2, dans lequel la numération cellulaire totale (TCC) élevée du patient dans le fluide de lavage bronchoalvéolaire (BALF) est réduite.

10. Un composé de formule (IA): 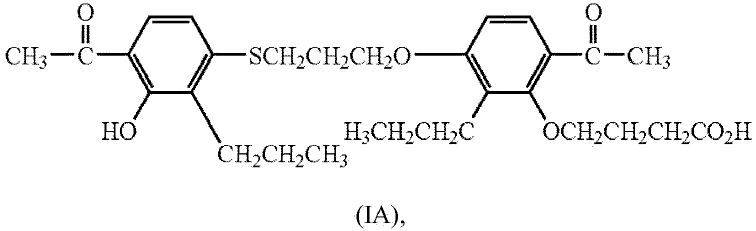 un ester de ce composé de formule (IA) ou un sel pharmaceutiquement acceptable de l'un ou l'autre d'entre eux, destiné à être utilisé dans une méthode de traitement d'un patient diagnostiqué comme présentant une IPF, la méthode comprenant l'administration au patient d'une quantité efficace du composé, de l'ester ou du sel en tant que seul ingrédient actif; dans lequel l'un ou l'autre des composés qui précèdent est fourni sous la forme d'une forme galénique solide comprenant des cristaux orthorhombiques, de préférence ledit composé est administré en une quantité d'environ 100 à environ 4000 mg/jour, divisée en une, deux ou trois portions.

11. Le composé, ester ou sel destiné à être utilisé selon la revendication 10, dans lequel la cicatrisation pulmonaire du patient est inhibée ou les niveaux élevés d'hydroxyproline dans les poumons du patient sont réduits et /ou inhibés, ou la densité pulmonaire élevée du patient est réduite, ou la numération cellulaire totale (TCC) élevée du patient dans le fluide de lavage broncho-alvéolaire (BALF) est réduit.

12. Le composé, ester ou sel destiné à être utilisé selon la revendication 10, dans lequel la forme posologique solide est administrée par voie orale.
